# EUROPÄISCHE PATENTSCHRIFT

(11) **EP 0 456 964 B1**
(45) Veröffentlichungstag und Bekanntmachung des Hinweises auf die Patenterteilung: **21.09.1994**
(21) Anmeldenummer: 91101747.3
(22) Anmeldetag: 08.02.1991
(51) Int. Cl.: A61B 17/064

(54) **Chirurgische Klammer**
Surgical staple
Agrafe chirurgicale

(30) Priorität: 08.05.1990 DE 4014653
(43) Veröffentlichungstag der Anmeldung: 21.11.1991
(73) Patentinhaber: Beiersdorf Aktiengesellschaft, 20245 Hamburg (DE)
(72) Erfinder: Heimerl, Albert, Dr., W-2000 Hamburg 65 (DE); Kartheus, Holger, W-2000 Hamburg 20 (DE)
(74) Vertreter: Vollmann, Heiko, Dipl.-Ing.

(56) Entgegenhaltungen:
- EP-A- 0 085 930
- EP-A- 0 180 820
- US-A- 4 321 002
- US-A- 4 399 810
- US-A- 4 583 670

## Beschreibung

Die Erfindung geht aus von einer chirurgischen Klammer mit einem geraden Steg und zwei Schenkein, die an ihren Enden mit Schneiden versehen sind und vom Steg ausgehend jeweils zunächst auf einem nach außen gekrümmten Abschnitt bogenförmig und anschließend auf einem Abschnitt gerade und nach innen auf die Symmetrieebene der Klammer gerichtet verlaufen.

Solche Klammern werden heute in zunehmendem Maß zum Verschließen operativer Hautwunden eingesetzt, so daß die traditionelle Methode des Wundverschlusses mit Nadel und Faden an Bedeutung verloren hat. Die bisher bekannten Wundklammern haben je nach ihrer Formgebung zwar gewisse Vorteile, aber auch eine Reihe von wesentlichen Nachteilen.

Bekannt sind sogenannte U-Klammern (EP-A-00 76 744, EP-A-0 284 345, US-A-3 643 851, US-A-4 179 057), die bezogen auf ihren unverformten Zustand eine einfache U-form haben und deshalb relativ preiswert in der Herstellung sind. Sie gewährleisten auch eine gute Adaptierung und Evertierung der Wundränder . Allerdings haben sie den gravierenden Nachteil, daß die Klammerschenkel und insbesondere auch deren Schneiden beim Erfassen der Wundränder und beim Implantieren traumatische Kanäle in die Haut und in das Gewebe reißen, was häufig zu Entzündungen in solchen Kanälen fuhrt. Bei der Entklammerung kommt noch der Nachteil hinzu, daß die Klammer beim Aufspreizen aufgrund einer ungünstigen Rückverformung erneut Gewebe zerstören und bereits verheilte Wundbereiche wieder aufreißen wird.

Dies gilt auch für Klammern (EP-A-0 180 820) der einleitend erwähnten Art, bei denen also die Schenkel vom geraden Steg ausgehend zunächst über eine nach außen gekrümmte Bogenstrecke verlaufen und anschließend einen geraden, nach innen auf die Symmetrieebene der Klammer gerichteten Verlauf haben. Solche Klammern haben zwar keine ideale U-Form, sie haben aber dennoch die gleichen Vor- und Nachteile wie U-Klammern.

Eine ähnliche Klammer wird in US-A-4583670 beschrieben, wobei die gerade verlaufenden Schenkelabschnitte unter einem Winkel zwischen 5° und 15° auf die Symmetrieebene der Klammer ausgerichtet sind.

Zu besseren Ergebnissen führen insofern die sogenannten D-Klammern, die im fertig verformten Zustand eine D-Form unabhängig davon haben, welche Form die Klammern vor der Verformung hatten (DE-A-2 625 991, DE-A-3 204 532, EP-A-0 085 930, US-A-1 910 688, US-A-4 321 002). Ein wesentliches gemeinsames Merkmal dieser D-Klammern ist eine bogenförmige Gestaltung der Klammerschenkel, die gewährleisten soll, daß beim Implantieren ein atraumatischer Stichkanal entsteht. Es hat sich aber herausgestellt, daß diesem möglichen Vorteil der D-Klammern ein großer Nachteil für die klinische Praxis gegenübersteht.

Für ein gutes Narbenergebnis ist es wichtig, daß die Wundheilung von der unteren Schicht (Stratum Germinativum) der Oberhaut (Epidermis) ausgeht und ohne Defektüberbrückung verläuft. Außerdem ist ein möglichst spannungsfreies Aneinanderlegen der Lederhaut (Corium) als mittlere Hautschicht und der untersten Hautschicht (Subcutis) erforderlich. Der Chirurg zieht deshalb zunächst die oberen Hautschichten an den Wundrändern keilförmig so auseinander, daß der Scheitel des Keiles im Bereich der eng zusammengefügten unteren Keimzellschicht (Stratum Germinativum) liegen wird. Diese auch Evertierung genannte Stellung wird dann mit einer Naht fixiert, da sie einige Tage lang nach dem Wundverschluß erhalten bleiben soll.

Es hat sich in der Praxis gezeigt, daß bei der Verklammerung einer Wunde mit D-Klammern die gewünschte Evertierung nicht erhalten bleibt, weil die bogenförmigen Klammerschenkel im Gewebe keine ausreichenden Haltekräfte aufbauen können und die Wundränder an den Klammerschenkeln nach oben gleiten werden. Solange beide Wundrandbereiche gleichmäßig an den Klammernschenkeln hochgleiten, resultiert daraus zwar ein für die Wundheilung nicht erwünschter, normalerweise aber noch tolerierbarer Effekt, der sich in einer relativ breiten Narbenbildung auswirkt.

Wenn es aber zu einer gegenläufigen oder unterschiedlich weiten Verschiebung der Wundränder an den beiden Schenkeln der Klammer kommt, werden sich Treppennarben ergeben, die dadurch besonders auffallen, daß beide Wundränder unter Bildung einer Stufe verheilen. Da in diesem Fall auch die drei oberen Hautschichten nicht korrespondierend aneinanderliegen, muß der Organismus selbst den Ausgleich zwischen den Hautschichten herstellen, wobei allerdings eine verhältnismäßig breite Narbe gebildet wird.

Man kann also feststellen, daß U-Klammern nur traumatisch implantierbar und entfernbar sind, im übrigen aber eine Evertierung der Wundränder erhalten können. Im Gegensatz hierzu sind D-Klammern weitgehend atraumatisch anwendbar, sie gewährleisten aber keine haltbare Evertierung der Wundränder.

Diese beiden Klammertypen haben im übrigen auch den Nachteil, daß sie keine Variationsmöglichkeiten für unterschiedliche Wundheilungsverläufe bieten. Es ist bekannt, daß insbesondere bei orthopädischen Operationen, wie etwa bei der Implantation von Hüftgelenk-Endoprothesen, die Wundränder durch das Instrumentarium mechanisch stark belastet werden und deshalb mehr anschwellen werden als Wundränder, die bei normalen Hautinzisionen entstehen. Deshalb werden die implantierten Klammerschenkel durch den Gewebedruck meist so stark auseinander gedrückt, daß an ihnen ungünstige Hebelkräfte entstehen. Hierbei kommt es vor, daß sich die Klammer und damit auch die Wunde teilweise wieder öffnet.

Es ist deshalb wünschenswert und Aufgabe der Erfindung, für solche Fälle eine Klammer anbieten zu können, die für eine variable Wundrandadaptierung geeignet ist, bei der die Klammer also auch in einem nicht vollständig verformten bzw. geschlossenen Zustand genügend Haltekräfte haben wird. Dabei soll die variable Wundrandadaptierung nicht nur bei Situationen großer Gewebeschwellungen, sondern auch in solchen Fällen Anwendung finden können, in denen unnötige Verletzungen der Unterhaut vermieden werden sollen. Dies gilt beispielsweise bei der Fixierung von Hauttransplantaten.

Weiterhin soll eine Klammer vorgeschlagen werden, mit der bei verhältnismäßig einfacher und damit kostengünstig herstellbarer Form eine weitgehend atraumatische, aber dennoch evertierungserhaltende Vereinigung von Wundrändern möglich ist.

Diese Aufgabe wird erfindungsgemäß durch die im Anspruch 1 angegebenen Merkmale gelöst.

Der Winkel α hat direkten Einfluß auf die Weite des Einstichkanals, da die kreisbogenförmigen Abschnitte der Schenkel beim Implantieren den geraden Abschnitten folgen und im Prinzip nur atraumatische Kanäle mit einem Durchmesser erzeugen würden, der dem des Klammermaterials entspricht. Je größer der Winkel α ist, desto größer wird der Einstichkanal, da dieser teilweise traumatisch entstehen wird. Mit größer werdendem Winkel α wird allerdings auch eine stärkere Verankerung der Klammerschenkel im Gewebe erzielt und besonders wirksam verhindert, daß die Wundränder an den Klammerschenkeln hochgleiten. Die Möglichkeit einer sicheren variablen Wundrandadaptierung, also die Verbindung von Wundrändern durch nur teilweise verformte Klammern, wird durch die Größe des Winkels α relativ wenig beeinflußt. Im übrigen ergeben sich umgekehrte Verhältnisse, wenn man von größeren Winkeln α auf kleinere übergeht. In allen Fällen ergibt sich eine "semitraumatische" Klammer, welche die vorerwähnten Vorteile der U-Klammern und D-Klammern hat, deren Nachteile aber weitestgehend ausschließt.

Eine CAD-Studie im Zusammenhang mit der möglichst gering zu haltenden Größe des Einstichkanals hat ergeben, daß der Winkel α im Bereich von 2,5° bis 10° liegen sollte, wobei sich ein Winkelbereich von etwa 7° bis 7,5° als besonders günstig herausgestellt hat.

Eine universell für einen großen Indikationsbereich geeignete Klammer, die auch den Bereich der plastischen Chirurgie mit abdeckt, wird im allgemeinen kleinere Winkel α in der Größenordnung von 4° bis 6° und vorzugsweise 5° haben.

In jedem Fall werden jedoch bei Anwendung der genannten Winkel und Winkelbereiche, die in ihrer absoluten Größe um ± 15 % schwanken können, vertretbar kleine Einstichkanäle unter weitestgehender Vermeidung traumatischer Einstiche und eine einwandfreie Fixierung und Evertierungserhaltung der Wundränder erreicht.

Der Klammersteg hat einen geraden Verlauf, wobei die kreisbogenförmigen Abschnitte der Klammerschenkel stetig auf diesen Steg übergehen können.

Im übrigen können die kreisbogenförmigen Abschnitte der Schenkel ebenfalls stetig auf die geraden Abschnitte übergehen, obwohl es auch möglich ist, daß die kreisbogenförmigen Abschnitte unter Bildung von Knickstellen auf die geraden Schenkelabschnitte übergehen können.

Die Erfindung wird nachfolgend anhand der in der Zeichnung dargestellten Ausführungsbeispiele näher erläutert. Es zeigt:

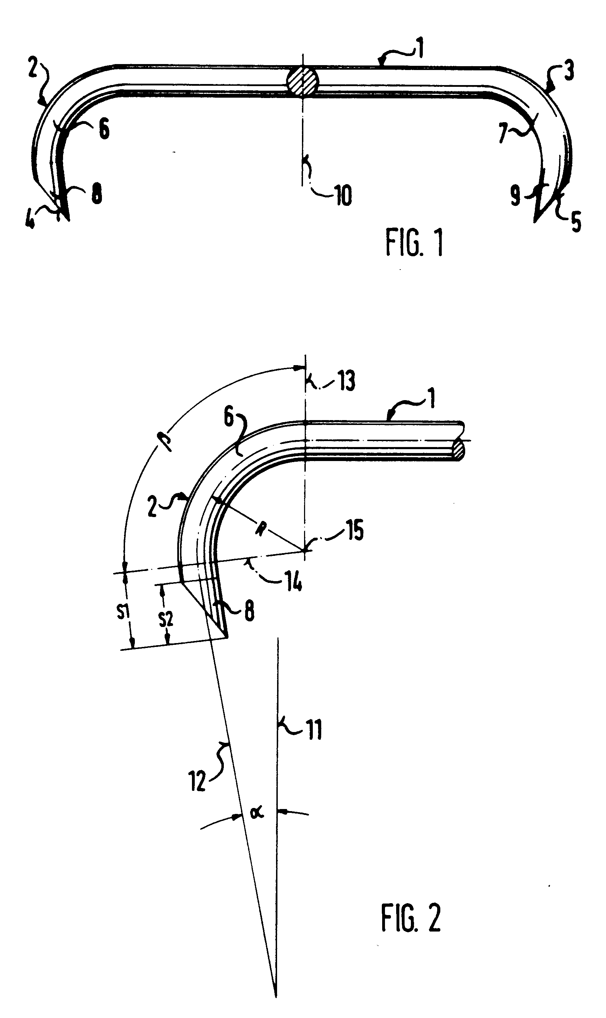

- Figur 1: Die Seitenansicht einer chirurgischen Klammer nach der Erfindung,
- Figur 2: einen Teil der in Figur 1 dargestellten Klammer in größerem Maßstab,
- Figur 3: die Klammer gemäß Figur 1 nach teilweiser Verformung,
- Figur 4: die Klammer gemäß Figur 1 in vollständig verformten Zustand und
- Figur 5: eine der Figur 2 entsprechende Darstellung, jedoch mit einem anderen Übergang des Schenkelbogens auf den geraden Schenkelabschnitt.

Die Klammer besteht aus einem geraden Steg 1, an dessen Enden sich zwei Schenkel 2, 3 anschließen, die an ihren Enden mit Schneiden 4, 5 versehen sind. Die Schenkel 2, 3 verlaufen vom Steg 1 ausgehend zunächst über einen Winkel β auf einem Kreisbogen mit dem Radius R und anschließend auf einer Strecke S1 gerade.

Die kreisbogenförmigen Abschnitte 6, 7 der beiden Klammernschenkel 2, 3 gehen stetig auf den Steg 1 und die geraden Abschnitte 8, 9 der Schenkel über, wobei diese Abschnitte unter einem Winkel α auf die Symmetrieebene 10 der Klammer bzw. auf eine hierzu parallel verschobene Ebene 11 ausgerichtet sind. Die gedachte Verlängerung 12 der Mittelachse der geraden Abschnitte 8, 9 wird also die genannten Ebenen unter einem Winkel α schneiden, für den die Beziehung 2,5° ≦ α ≦ 10° gelten soll. Engere und für bestimmte Anwendungsfälle vorteilhafte Winkelbereiche wurden schon vorher diskutiert.

Der Winkel β liegt zwischen einer Normalen 13 und einer Linie 14, die durch den Übergang zwischen den Schenkelabschnitten 6, 7 und 8, 9 verläuft. Die gedachte Normale 13 steht auf dem Radiuspunkt 15 und schneidet den Steg 1 senkrecht. Sie wird die Klammer im übrigen dort schneiden, wo die kreisbogenförmigen Abschnitte 6, 7 auf den Steg 1 übergehen, sofern dieser Übergang stetig ist.

Im übrigen sollte der Winkel β in folgender Größenordnung liegen, nämlich 80° ≦ β ≦ 100°. Insofern wird zu beachten sein, daß dieser Winkel auch vom jeweils gewählten Winkel α in gewissem Maße abhängen wird. Eine zweckmäßige Anpassung und Abstimmung hinsichtlich der beiden Winkel α und β kann auch erreicht werden, indem man gemäß Figur 5 die kreisbogenförmigen Abschnitte 6, 7 jeweils unter Bildung einer Knickstelle 16 auf die geraden Abschnitte 8, 9 übergehen läßt. Diese Möglichkeit kommt insbesondere auch bei Winkeln β in Betracht, die unter 90° oder nur etwas über 90° betragen, wobei dann der Winkel α einfach durch mehr oder weniger starkes Abknicken der geraden Schenkelabschnitte erreicht werden kann.

Bei den gezeigten Ausführungsbeispielen gehören zu den geraden über die Strecke S1 verlaufenden Abschnitte 8, 9 die schrägen Schneiden 4, 5 und Teile des Klammermaterials mit vollem Querschnitt. Es ist allerdings auch möglich, die geraden Abschnitte der Klammerschenkel kürzer auszubilden und beispeilsweise nur über eine Strecke S2 verlaufen zu lassen, die der axial gemessenen Länge der Schneiden entspricht.

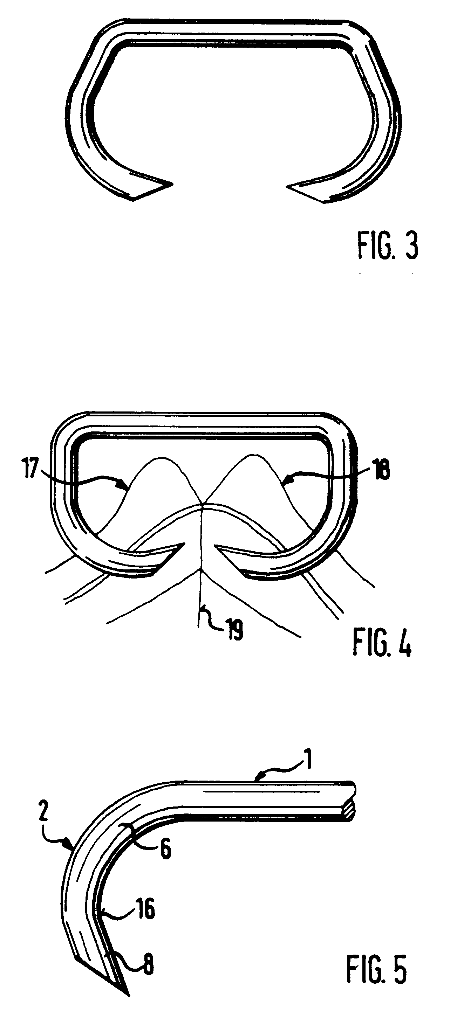

Geräte zum Setzen von chirurgischen Klammern sind allgemein bekannt und brauchen deshalb nicht weiter beschrieben zu werden. Bei entsprechender Auslegung und Anwendung dieser Geräte lassen sich die Klammern auch gezielt in eine nur teilweise geschlossene Form bringen, wie es die Figur 3 zeigt und was beispielsweise für eine variable Wundrandadaptierung oder für Fixierungen von Hauttransplantaten in Betracht kommt.

In der Figur 4 ist die Klammer in geschlossenem und vollständig implantierten Zustand gezeigt. Man erkennt an dieser Darstellung auch deutlich, daß die schematisch angedeuteten Wundrandbereiche 17, 18 evertiert sind und daß die geraden Abschnitte der Klammerschenkel das über ihnen befindliche Gewebe abstützen und die Evertierung erhalten werden, ohne daß es wie bei etwa vollständig kreisbogenförmigen Klammernschenkeln dazu kommen kann, daß Gewebe an den Schenkeln hochgleitet und dabei die Evertierung verschwindet.

## Patentansprüche

1. Chirurgische Klammer mit einem geraden Steg (1) und zwei Schenkeln (2, 3), die an ihren Enden mit Schneiden (4, 5) versehen sind und vom Steg ausgehend jeweils zunächst auf einem nach außen gekrümmten Abschnitt (6, 7) bogenförmig und anschließend auf einem Abschnitt (8, 9) gerade und nach innen auf die Symmetrieebene (10) der Klammer gerichtet verlaufen, wobei die gerade verlaufenden Schenkelabschnitte (8, 9) unter einem Winkel 2,5° ≦ α ≦ 10° auf die Symmetrieebene (10) der Klammer ausgerichtet sind und für den Winkel β, über den die bogenförmigen Abschnitte (6, 7) der Schenkel jeweils in Form eines einzelnen Kreisbogens verlaufen und der zu messen ist zwischen einer durch den Radiuspunkt (15) des jeweiligen Kreisbogens und den Klammersteg (1) verlaufenden Normalen (13) einerseits und dem Übergang des Kreisbogens auf den gerade verlaufenden Abschnitt (8, 9) des jeweiligen Schenkels andererseits, folgende Beziehung gilt, nämlich 80° ≦ β ≦ 100°.

2. Klammer nach Anspruch 1, gekennzeichnet durch einen Winkel α, für den die Beziehung 7° ≦ α ≦ 7,5° gilt.

3. Klammer nach Anspruch 1, gekennzeichnet durch einen Winkel α, für den die Beziehung 4° ≦ α ≦ 6°, insbesondere α = 5° gilt.

4. Klammer nach einem der Ansprüche 1 bis 3, dadurch gekennzeichnet, daß die kreisbogenförmigen Abschnitte (6, 7) der Schenkel (2, 4) stetig auf den Steg (1) übergehen.

5. Klammer nach einem der Ansprüche 1 bis 4, dadurch gekennzeichnet, daß die kreisbogenförmigen Abschnitte (6, 7) der Schenkel (2, 3) stetig auf die geraden Schenkelabschnitte (8, 9) übergehen.

6. Klammer nach einem der Ansprüche 1 bis 4, dadurch gekennzeichnet, daß die kreisbogenförmigen Abschnitte (6, 7) der Schenkel (2, 3) unter Bildung von Knickstellen (16) auf die geraden Schenkelabschnitte (8, 9) übergehen.

## Claims

1. A surgical clip with a straight cross-piece (1) and two shanks (2,3), which are provided at their ends with cutting edges (4,5) and which, proceeding from the cross-piece, in each case run firstly on an outwardly curved section (6,7) in a curved shape and then on a section (8,9) run straight and directed inwardly towards the plane of symmetry (10) of the clip, in which the shank sections (8,9) which run straight are aligned at an angle 2.5° ≦ α ≦ 10° to the plane of symmetry (10) of the clip and for the angle β, over which the curved sections (6,7) of the shanks in each case run in the form of a single circular arc and which is to be measured between a normal (13) running through the radius point (15) of the respective circular arc and the clip cross-piece (1) on the one hand and the transition of the circular arc onto the straight section (8,9) of the respective shank on the other hand, the following relationship applies, namely 80° ≦ β ≦ 100°.

2. A clip according to Claim 1, characterised by an angle α, for which the relationship 7° ≦ α ≦ 7.5° applies.

3. A clip according to Claim 1, characterised by an angle α, for which the relationship 4° ≦ α ≦ 6° applies, in particular α = 5°.

4. A clip according to one of Claims 1 to 3, characterised in that the sections (6,7) of the shanks (2,4), in the form of a circular arc, continuously pass over onto the cross-piece (1).

5. A clip according to one of Claims 1 to 4, characterised in that the sections (6,7) of the shanks (2,3), in the form of a circular arc, continuously pass over onto the straight shank sections (8,9).

6. A clip according to one of Claims 1 to 4, characterised in that the sections (6,7) of the shanks (2,3), in the form of a circular arc, pass over onto the straight shank sections (8,9) with the formation of kinks (16).

## Revendications

1. Agrafe chirurgicale comportant un dos rectiligne (1) et deux ailes (2, 3) qui sont pourvues, à leurs extrémités, d'arêtes de coupe (4, 5), et qui s'étendent chacune, tout d'abord sur un tronçon (6, 7) courbé vers l'extérieur selon une forme d'arc de cercle, et consécutivement sur un tronçon (8, 9) de manière rectiligne et vers l'intérieur en direction du plan de symétrie (10) de l'agrafe, les tronçons d'aile (8, 9) qui s'étendent de manière rectiligne étant orientés de manière à former un angle 2,5° ≦ α ≦ 10° par rapport au plan de symétrie (10) de l'agrafe, et la relation 80° ≦ β ≦ 100° étant réalisée pour l'angle β sur lequel s'étendent les tronçons (6, 7) des ailes, en forme d'arc, chacun sous la forme d'un arc de cercle individuel, l'angle β étant mesuré entre une normale (13) passant par le centre (15) de l'arc de cercle considéré et le dos (1) de l'agrafe d'une part, et le raccordement de l'arc de cercle au tronçon (8, 9) rectiligne de l'aile considérée, d'autre part.

2. Agrafe selon la revendication 1, caractérisée par un angle α pour lequel est valable la relation 7° ≦ α ≦ 7,5°.

3. Agrafe selon la revendication 1, caractérisée par un angle α pour lequel est valable la relation 4° ≦ α ≦ 6°, et notamment α = 5°.

4. Agrafe selon l'une des revendications 1 à 3, caractérisée en ce que les tronçons (6, 7) en forme d'arc de cercle des ailes (2, 3), se raccordent de manière continue sur le dos (1).

5. Agrafe selon l'une des revendications 1 à 4, caractérisée en ce que les tronçons (6, 7) en forme d'arc de cercle des ailes (2, 3), se raccordent de manière continue sur les tronçons d'aile (8, 9) rectilignes.

6. Agrafe selon l'une des revendications 1 à 4, caractérisée en ce que les tronçons (6, 7) en forme d'arc de cercle des ailes (2, 3), se raccordent aux tronçons d'aile (8, 9) rectilignes, avec formation d'emplacements d'infléchissement (16).
